# EUROPÄISCHE PATENTANMELDUNG

(11) **EP 1 840 217 A2**
(43) Veröffentlichungstag der Anmeldung: **03.10.2007**
(21) Anmeldenummer: 07001667.0
(22) Anmeldetag: 26.01.2007
(51) Int. Cl.: C12P 21/02, C12N 15/79

(54) **Verfahren zur Genexpression**

(30) Priorität: 30.03.2006 DE 102006015321
(71) Anmelder: RiNA Netzwerk RNA-Technologien GmbH, 14195 Berlin (DE)
(72) Erfinder: Merk, Helmut, Dr. c/o RiNA Netzwerk, 14195 Berlin (DE); Stiege, Wolfgang, Dr. c/o RiNA Netzwerk, 14195 Berlin (DE); Kubick, Stefan, Dr. c/o RiNA Netzwerk, 14195 Berlin (DE)
(74) Vertreter: Jungblut, Bernhard Jakob

(57) **Zusammenfassung**

Die Erfindung betrifft ein Verfahren zur Genexpression in einem zellfreien Translationssystem, wobei die Reaktionslösung eine RNA-Matrize mit einer Gensequenz, die für ein zu exprimierendes Expressionsprodukt kodiert, sowie ein Translationssystem aus eukaryontischen Zellen, enthält, wobei die Reaktionslösung inkubiert wird, und wobei das Expressionsprodukt optional von der Reaktionslösung abgetrennt wird, dadurch gekennzeichnet, dass die RNA-Matrize, in 5'- 3'- Richtung betrachtet, eine Shine Dalgarno Sequenz, hieran angeschlossen eine erste Spacer Sequenz und hieran angeschlossen die Gensequenz enthält.

## Beschreibung

### Gebiet der Erfindung

Die Erfindung betrifft ein Verfahren zur Genexpression in einem zellfreien Translationssystem, wobei die Reaktionslösung eine RNA-Matrize mit einer Gensequenz, die für ein zu exprimierendes Expressionsprodukt kodiert, sowie ein Translationssystem aus eukaryontischen Zellen, enthält, wobei die Reaktionslösung inkubiert wird, und wobei das Expressionsprodukt optional von der Reaktionslösung abgetrennt wird. Die Erfindung betrifft des Weiteren ein Kit zur Durchführung eines solchen Verfahrens sowie eine RNA zur Verwendung in einem solchen Verfahren.

### Stand der Technik und Hintergrund der Erfindung

Verfahren zur zellfreien Expression von Proteinen sind beispielsweise aus den Literaturstellen EP 0312 617 B1, EP 0401 369 B1 und EP 0 593 757 B1 bekannt.

Demgemäß werden die für eine Transkription und/oder Translation notwendigen Komponenten neben einem für ein gewünschtes Protein kodierenden Nukleinsäurestrang in einem Reaktionsgefäß inkubiert und nach der Expression die Polypeptide/Proteine aus der Reaktionslösung isoliert.

Sowohl die für die Transkription, als auch die für die Translation notwendigen Komponenten lassen sich leicht aus den Überstanden pro- oder eukaryontischer Zelllysate nach beispielsweise einer 30000 x g Zentrifugation gewinnen. Dieser sogenannte S-30 Extrakt enthält alle für die Transkription und Translation notwendigen Komponenten.

Die Expression erfolgt typischerweise bei 27°C oder 37°C, kann aber auch bei Temperaturen von 17°C bis 45°C erfolgen. Die Anpassung der Temperatur empfiehlt sich insbesondere bei der Expression von Proteinen, in denen eine komplexe Sekundär-/Tertiärstruktur ausgebildet werden soll. Durch Absenken der Temperatur kann die Syntheserate gesenkt und somit den Proteinen die Möglichkeit gegeben werden, sich richtig zu falten, um ein funktionstüchtiges/aktives Protein zu erhalten.

In dem im Dokument EP 0312 617 B1 offenbarten Verfahren zur zellfreien Expression von Proteinen wird der für das Protein kodierende Nukleinsäurestrang als mRNA der Reaktionslösung zugegeben. Damit müssen für die Herstellung von Polypeptiden in dem zellfreien System nur die für die Translation notwendigen Komponenten des Translationsapparates, insbesondere Ribosomen, Initiations-, Elongations-, Freisetzungsfaktoren und Aminoacyl-tRNA-Synthetase, sowie Aminosäuren und als Energie liefernde Substanzen ATP und GTP in ein Reaktionsgefäß gegeben werden. Bei der anschließenden Polypeptid-/Proteinsynthese kommt es neben der Bildung von Polypeptiden/Proteinen auch zur Bildung von niedermolekularen Substanzen, wie ADP, AMP, GDP, GMP und anorganischen Phosphaten unter Verbrauch der Energie liefernden Substanzen ATP und GTP und von Aminosäuren. Zur Aufrechterhaltung der Reaktion können die während der Translation verbrauchten Substanzen während der Translation herausgeführt und gleichzeitig die Energie liefernden Substanzen und die Aminosäuren zur Erhaltung der Ausgangskonzentration eingeführt werden.

Das Dokument EP 0401 369 B1 offenbart ein Verfahren, bei dem die für das Protein kodierende Nukleinsäure als mRNA oder DNA der Reaktionslösung zugegeben werden kann. Letzteres hat den Vorteil, daß DNA wesentlich stabiler als mRNA ist, und der notwendige (ggf. separat durchgeführte Umschreibeprozess der DNA in RNA vor der Reaktion nicht notwendig ist, sondern gleich die DNA, z.B. als Vektor oder lineares Konstrukt eingesetzt werden kann. Durch den Einsatz der DNA muß das zellfreie Expressionssystem neben den obengenannten Translationsfaktoren noch die für die Transkription der DNA in RNA notwendigen Transkriptions-faktoren, wie z.B. RNA-Polymerase, -Faktor und Rho-Protein und die Nukleotide ATP, UTP, GTP und CTP enthalten. Auch hier können die während der Transkription/Translation verbrauchten niedermolekularen Substanzen, wie ADP, AMP, GDP, GMP und anorganischen Phosphate während der Translation herausgeführt werden und gleichzeitig die Energie liefernde Substanzen, Nukleotide und die Aminosäuren zur Erhaltung der Ausgangskonzentration eingeführt werden.

Weitere Verfahren zur Genexpression sind beispielsweise aus den Literaturstellen DE 101 37 792 A und DE 10 2004 032 460 A. Ein Verfahren zur Herstellung eines Lysats für die Genexpression ist aus der Literaturstelle DE 103 36 705 A bekannt.

Die Shine Dalgarno Sequenz (Shine, J., Dalgarno, L., Nature, 254(5495):34-38 (1975)) ist eine Teilsequenz in der mRNA von Prokaryoten, die von den Ribosomen erkannt wird und damit den Startpunkt der Translation markiert. Sie liegt typischerweise 5'-seitig des ersten codierenden AUG und besteht überwiegend aus Purinen. Die Sequenz ist speziestypisch und kann für verschiedene Spezies den öffentlich zugänglichen Gendatenbanken entnommen werden. Demgegenüber weist die RNA von Eukaryonten als Initialisierungssequenz typischerweise die Kozak Sequenz auf. Im Insektenzellsystem hat die Kozak Sequenz dagegen sehr geringe Bedeutung. Hier wird die Translationseffizienz mit Hilfe der 5' -UTR des Polyhedringens aus dem Baculovirus *Autographa californica* sehr stark gesteigert (Raming K. Krieger J., Strotmann J, Boekhoff I, Kubick S, Baumstark C and Breer H, 1993, Cloning and expression of odorant receptors, Nature 361, 353-356).

Aus dem Vorstehenden ergibt sich, dass die einzusetzende RNA bzw. die DNA, die zu der gewünschten RNA transkribiert wird, nach Maßgabe des Systems, eukaryontisch oder prokaryontisch, unterschiedlich sein muß. Verschiedene Hersteller von Proteinen und dergleichen bevorzugen aus unterschiedlichen Gründen verschiedene Systeme. Hieraus folgt, dass für die Synthese eines spezifischen Genprodukts, beispielsweise eines bestimmten Proteins, zwei verschiedene RNA bzw. DNA hergestellt werden müssen, wenn die Expression sowohl in eukaryontischen als auch in prokaryontischen Systemen mit produktionstechnisch interessanten Ausbeuten möglich sein soll, und zwar obwohl die gleiche Gensequenz zum Einsatz kommt. Dies ist aufwändig.

### Technisches Problem der Erfindung

Der Erfindung liegt das technische Problem zu Grunde, ein Verfahren zur zellfreien Genexpression in einem eukaryontischen System anzugeben, in welchem eine RNA verwendet werden kann, die ohne Änderung auch in einem prokaryontischen System einsetzbar ist.

### Grundzüge der Erfindung und bevorzugte Ausführungsformen

Zur Lösung dieses technischen Problems lehrt die Erfindung ein Verfahren der eingangs genannten Art, welche dadurch gekennzeichnet ist, dass die (m)RNA-Matrize, in 5'- 3'-Richtung betrachtet, eine Shine Dalgarno Sequenz, hieran vorzugsweise unmittelbar angeschlossen eine erste Spacer Sequenz und hieran vorzugsweise unmittelbar angeschlossen die Gensequenz enthält.

Die Erfindung beruht auf der überraschenden Erkenntnis, dass eine Genexpression sowohl in einem prokaryontischen System als auch in einem eukaryontischen System mit einer RNA möglich ist, auch wenn sie keine Kozak Sequenz, sondern vielmehr die für Prokaryoten typische Shine Dalgarno Sequenz als Initialisierungssequenz enthält.

Mit der Erfindung wird erreicht, dass mit einer einzigen mRNA (bzw. einer hierfür codierenden DNA) ein definiertes Genprodukt nach Wahl eines Anwenders entweder in einem prokaryontischen System oder in einem eukaryontischen System exprimiert werden kann. Dadurch wird die Konstruktion verschiedener RNA bzw. DNA für das gleiche Gen, jedoch verschiedene Systeme entbehrlich, man erhält gleichsam ein Universalprodukt für beide Systeme. Hierbei ist es möglich, grundsätzlich alle Gene einzusetzen, die sich in dem betreffenden System exprimieren lassen. Sequenzen gewünschter Gene, als DNA oder (m)RNA, können den öffentlich zugänglichen Gendatenbanken entnommen werden.

Im Rahmen der Erfindung wurde festgestellt, dass die mRNA für eine Genexpression in einem eukaryontischen System nicht notwendigerweise die Eukaryonten-typischen Elemente 5'-terminales cap, Kozak Sequenz und 3'-terminaler Poly(A) Schwanz aufweisen muss, wenn statt dessen eines oder mehrere der folgenden Prokaroyten-typischen Elemente eingerichtet ist: 5'-UTR, Sekundärstruktur am 5'-Ende (Hairpin), Shine Dalgarno Sequenz und/oder von Bakteriophagen abstammender Transkriptionsterminator. Insbesondere kann auch an Stelle eines cap ein Biotinrest am 5'-terminalen Ende angeordnet sein.

Cap steht für 7-Methyl-GTP und tritt in der Natur in 3 verschiedenen Formen auf: Alle Caps enthalten 7-Methylguanylat, das über eine Triphosphatbindung an die Ribose am 5'-Ende geknüpft ist. Cap 0 besitzt keine methylierte Ribose, Cap 1 eine, und in Cap 2 sind zwei Ribosen am C2 Sauerstoffatom methyliert. Das7-Methyl-GTP wird für die Lokalisierung der mRNA am Ribosom benötigt. Poly (A) schützt das 3'-Ende vor Abbau durch Exonucleasen.

In einer bevorzugten Ausführungsform sind im 5'- Bereich der RNA-Matrize vor der Shine Dalgarno Sequenz kein ATG und/oder kein AUG mit open reading frame angeordnet.

Grundsätzlich kann das 5'-terminale Ende der RNA unmodifiziert sein. Eine Verbesserung der Ausbeute wird jedoch erreicht, wenn das 5'-terminale Ende der Nukleotidsequenz der RNA-Matrize aus einer cap-Struktur gebildet ist oder einen Biotinrest trägt.

Zwischen dem 5'-terminalen Ende der RNA-Matrize und der Shine Dalgarno Sequenz kann eine Enhancersequenz, insbesondere eine von Bakteriophagen abstammende 5'-nicht-translatierte Sequenz (5'-UTR) angeordnet sein. Dies wird sich empfehlen, um insbesondere eine Translationsrate zu erhöhen, insbesondere in Prokaryonten. Überraschenderweise stört die Enhancersequenz zudem in eukaryontischen Systemen nicht.

Es kann vorgesehen sein, dass zwischen dem 5'-terminalen Ende der RNA-Matrize und der Enhancersequenz eine Hairpinstruktur angeordnet ist. Diese ist auch in eukaryontischen Systemen zum Schutz gegen Abbau wirksam. Natürliche, effizient translatierte Eukaryonten-typische mRNAs weisen jedoch keine starken Sekundärstrukturen an ihrem 5'-Ende auf, da sonst die Translationsinitiation aufgrund einer schlechten Zugänglichkeit des Caps für Initiationsfaktoren inhibiert ist (Hershey JWB and Merrick WC, 2000, The pathway and mechanism of initiation of protein synthesis. S. 33-88, in: Translational control of gene expression. eds. Sonenberg N, Hershey JWB and Mathews MB, Cold Spring Harbour Laboratory Press, New York). Entsprechendes gilt, wenn das 3'-terminale Ende der RNA-Matrize durch einen von Bakteriophagen abstammenden Transkriptionsterminator, insbesondere durch eine Hairpinstruktur, gebildet wird.

Die erste Spacersequenz kann aus 3 bis 20, vorzugsweise 5 bis 15, Nukleotiden, vorzugsweise Pyrimidin-reichen Nukleotiden, jedoch keine Purinfolge, gebildet sein. Zwischen der Gensequenz und dem 3'-terminalen Ende der RNA-Matrize kann eine zweite Spacersequenz angeordnet sein, welche vorzugsweise eine Länge von 10 bis 50, insbesondere 20 bis 40, Nukleotiden aufweist.

Im Einzelnen kann die RNA-Matrize vom 5'-terminalen Ende beginnend die folgenden optional unmittelbar aneinander anschließenden Strukturelemente aufweisen: cap oder Biotin, optional Hairpin, optional Enhancersequenz, Shine Dalgarno Sequenz, erste Spacersequenz, Gensequenz, optional zweite Spacersequenz, Transkriptionsterminator.

Erfindungsgemäße RNA ist beispielsweise in Translationssystemen einsetzbar, die aus den folgenden Zellen bzw. Zelllinien als Lysate gewonnen wurden: BHK21 (Hamster), MOLT-4 (Human), MOPC 21 (Maus), RPMI 8226 (Human), Jurkat FHCRC (Human), HL60 (Human) HEK 293 (Human), CHO (Hamster), HeLa (Human), PC12 (Ratte), Sf 9 (Insekt), Sf21 (Insekt), COS-1 (Affe), COS-7 (Affe), D2 (Drosophila), NIH 3T3 (Maus), Tn5 B1-4 (Insekt), und Tn 368 (Insekt). Auch sind Lysate aus tierischem und pflanzlichem Ursprung einsetzbar, wie beispielsweise Kaninchen Retikulozyten Lysat oder Weizenkeim Lysat. Bevorzugte Lysate werden aus Insektenzellen gewonnen. Die geeigneten Shine Dalgarno Sequenzen und Enhancersequenzen lassen sich unschwer den öffentlichen Gendatenbanken zu der gewählten Insektenspezies entnehmen.

Die Erfindung betrifft des Weiteren ein Kit für die Genexpression in einem zellfreien Translationssystem, wahlweise eukaryontisch oder prokaryontisch, enthaltend die folgenden Komponenten: a) Transkriptions- und/oder Translationssystem aus eukaryontischen Zellen, insbesondere Insektenzellen, b) RNA-Matrize, die, in 5'- 3'- Richtung betrachtet, eine Shine Dalgarno Sequenz, hieran vorzugsweise unmittelbar angeschlossen eine erste Spacer Sequenz und hieran vorzugsweise unmittelbar angeschlossen eine Gensequenz enthält, oder DNA codierend für eine solche RNA. Das Kit kann zusätzlich auch ein Transkriptions- und/oder Translationssystem aus prokaryontischen Zellen enthalten, so dass ein Anwender zwischen den Systemen wählen kann. Wenn DNA eingesetzt wird, versteht es sich, dass die jeweiligen Systeme die notwendigen Transkriptionsfaktoren enthalten.

Die Erfindung betrifft des Weiteren eine (m)RNA enthaltend eine sterische Schutzgruppe (nicht jedoch ein cap) am 5'-terminalen Ende, eine Shine Dalgarno Sequenz und am 3'-Ende der Shine Dalgarno Sequenz eine Gensequenz. Als sterische Schutzgruppe kommt beispielsweise eine der folgenden Strukturen in Frage: Biotin Digoxigenin, Fluorophore wie Fluorescein, Cy3, Cy5, Bodipy, Alexa, Atto; Gp₄G; Ap₃G; G; m⁷Gp₄G; m⁷Gp₃m⁷G; m⁷Gp₄m⁷G; benz⁷Gp₃G; benz⁷Gp4_{G}; benz⁷, 3' OMeGp₄G; et ⁷Gp₃G; m⁷, 3' OMeGp₃G; m⁷, 3' OMeGp₄G; m⁷, 2'OMeGp₄G; m⁷Gp₅G; m⁷, 3' OMeGp₅G; m⁷, 2' deoxyGp₃G; M⁷, 2'deoxyGP₄G; m⁷GpCH₂ppG; m⁷GppCH₂pG; Gp₃G; m⁷, 3' OMeGpCH₂ppG; m⁷, 3' OMeGppCH₂pG; m⁷,2'OMeGppCH₂pG; m⁷GpCH₂ppm⁷G.

Von der Erfindung mit umfasst sind auch RNA-Hybride mit Oligomeren aus DNA, RNA, PNA und modifizierte Formen hiervon, die auch mit weiteren chemischen Gruppen wie s.o. modifiziert sein können.

Die Erfindung betrifft schließlich eine DNA codierend für eine erfindungsgemäße RNA.

In Bezug auf die weiteren Merkmale der RNA bzw. DNA, für sich genommen oder im Kit, wird auf die vorstehenden Erläuterungen verwiesen.

Im Folgenden wird die Erfindung anhand von lediglich Ausführungsformen darstellenden Beispielen näher erläutert.

### Beispiel 1: erfindungsgemäße mRNA

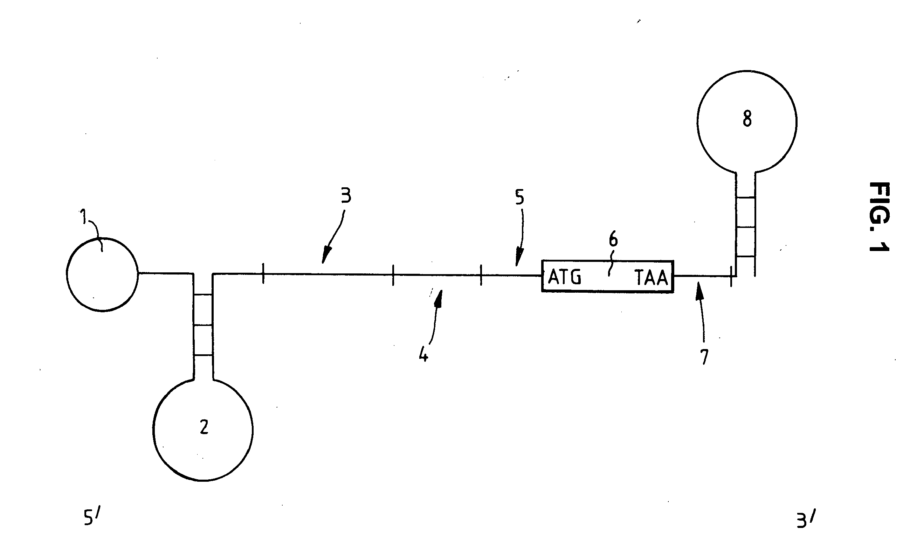

Die Figur 1 zeigt den prinzipiellen Aufbau einer erfindungsgemäßen mRNA. Man erkennt zunächst einen Biotinrest 1. Statt dessen kann auch eine andere sterische Schutzgruppe, ggf. auch ein 7-Methyl-Guanosin-Gruppe als cap, eingerichtet sein. Hieran schließen sich eine Hairpinstruktur 2 und eine Phage Enhancersequenz 3 an. Es folgt die Shine Dalgarno Sequenz 4. Über eine erste Spacersequenz 5 bestehend aus 5 bis 8 Nukleotiden, die überwiegend Pyrimidinbasen sind, ist die für das zu exprimierende Gen codierende Gensequenz 6 angeschlossen. Es folgt eine zweite Spacersequenz 7 mit 20 bis 40 Nukleotiden, die praktisch beliebig sein können. Am 3'-terminalen Ende ist wiederum eine Hairpinstruktur 8 angeordnet.

Die Auswahl der Shine Dalgarno Sequenz erfolgt nach Maßgabe der eukaryontischen Zellspezies, welche die Basis für das Translationssystem bilden.

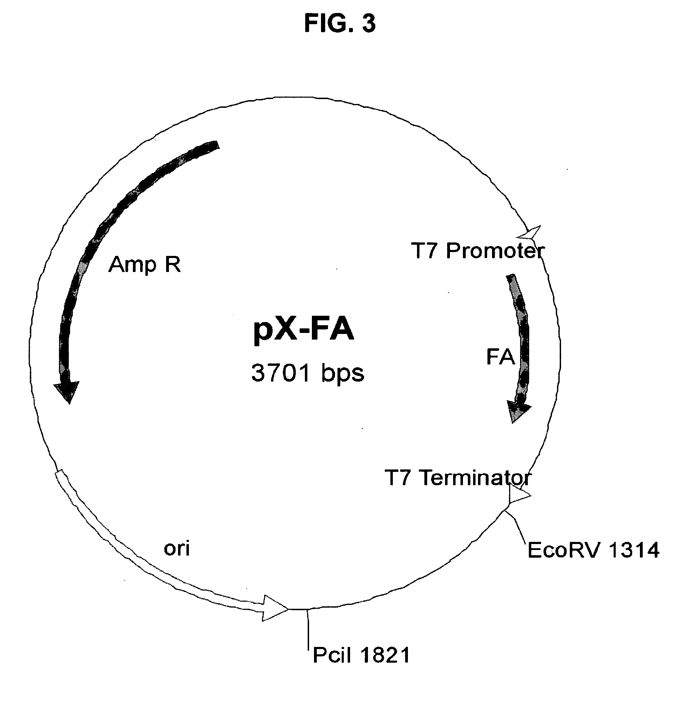

Eine konkrete Sequenz, die beispielsweise für die Expression des Proteins "Fettsäure Bindendes Protein aus Rinderherz" in einem Expressionssystem auf Basis von Zellen der Insektenspezies geeignet ist, ist lediglich beispielhaft in Sequenz Seq.-ID 1 angegeben. Es ist transkribiert von dem Plasmid pXFA, dessen Vektorkarte in der Figur 3 und dessen Sequenz in Seq.-ID 2 angegeben sind. Es versteht sich, dass beliebige andere Gensequenzen, Shine Dalgarno Sequenzen usw. eingesetzt werden können, die lediglich nach Maßgabe des gewünschten Proteins und des gewünschten Expressionssystems auszuwählen sind.

### Beispiel 2: Herstellung der mRNA nach Beispiel 1

Das Plasmid pXFA (Fig. 3, Seq.-ID 2) wurde mit den Restriktionsenzymen EcoRV und PciI (NEB) nach Herstellerangaben verdaut. Der hieraus resultierende Restvektor (3196 Bp) wurde elektrophoretisch im Agarosegel von dem ausgeschnittenen Plasmidfragment und unvollständig verdautem Plasmid getrennt. Das Gelstück mit dem Restvektor wurde ausgeschnitten, hieraus die DNA mittels Gelelution (High Pure PCR Product Purification Kit, Roche) entsprechend Herstellerangaben aufgereinigt und die DNA-Konzentration photometrisch anhand der Absorption von Licht bei 260 nm bestimmt.

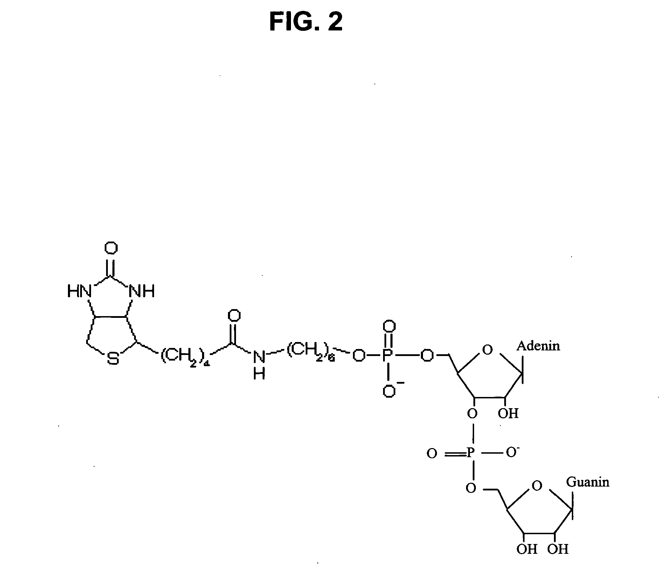

Die DNA wurde mit dem EasyXpress Protein Synthesis Insect Kit (Qiagen) mit einer Ausnahme nach Herstellerangaben in RNA transkribiert: anstelle des NTP-Mix des Kits wurden folgende Komponenten mit den auf die Transkriptionsreaktion bezogenen Endkonzentrationen eingesetzt: 3,75 mM jeweils ATP, CTP und UTP, 1,5 mM GTP (alle Roche) und 2 mM Biotin-ApG (Auftragssynthese Noxxon AG, Berlin, Deutschland, Fig. 2).

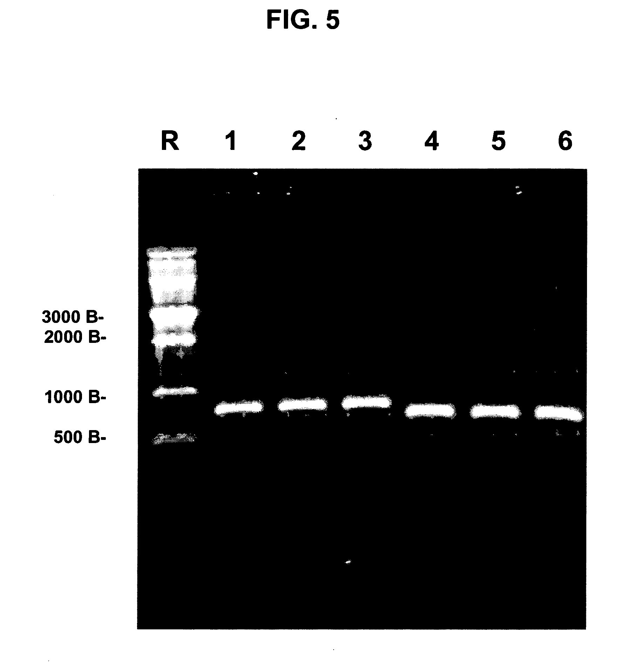

Im Anschluß an die Transkription wurde der Reaktionsansatz zur Eliminierung der DNA mit 0,5 µl 10 U/µl RNase-freier DNaseI (Roche) versetzt und 30 min bei 37°C inkubiert. Danach wurde der Reaktionsansatz mit dem Reinigungssystem des Kits aufgereinigt. Die RNA-Konzentration des gereinigten Transkriptionsansatzes wurde photometrisch anhand der Absorption von Licht bei 260 nm bestimmt und gelelektrophoretisch analysiert (Fig. 5).

Es wurde eine weitere mRNA wie vorstehend angegeben hergestellt mit folgender Ausnahme: anstelle des NTP-Mix des Kits wurden folgende Komponenten mit den auf die Transkriptionsreaktion bezogenen Endkonzentrationen eingesetzt: 3,75 mM jeweils ATP, CTP und UTP, 1,5 mM GTP (alle Roche) und 0,5 mM P1,P3-Di(guanosine-5')triphosphate (Katalog-Nr. D1012, Sigma). Die mRNA wurde wie in Beispiel 2 angegeben weiterverarbeitet und gelelektrophoretisch analysiert (Fig. 5).

### Beispiel 3: Expression der Proteine aus Beispiel 2 in einem eukaryontischen System.

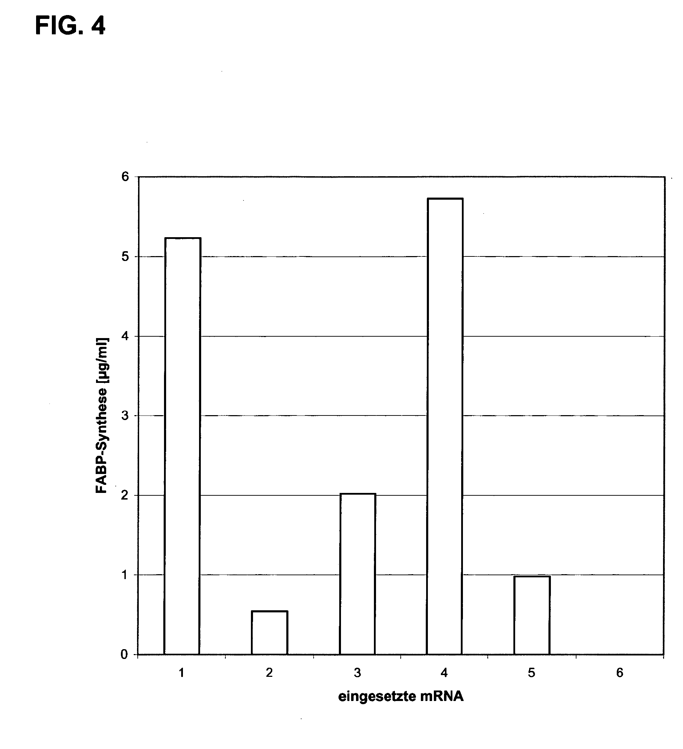

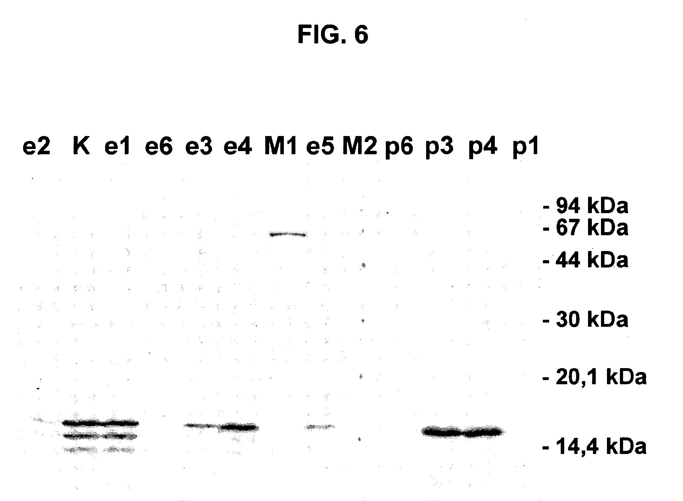

Die erste in Beispiel 2 beschriebene mRNA wurde, bezogen auf die Translationsreaktion, in einer Endkonzentration von 600 nM für die nach Herstellerangaben durchgeführte zellfreie Translation mit dem EasyXpress Protein Synthesis Insect Kit (Katalog-Nr. 32552, Qiagen) eingesetzt. Zusätzlich wurde für die Translation ¹⁴C-markiertes Valin eingesetzt, so dass sich eine molare Aktivität von 80 dpm/pmol eingestellt hat. Die Proteinausbeuten wurden anhand des Einbaus der radioaktiv markierten Aminosäure in heißer Trichloressigsäure unlöslichem Reaktionsprodukt und Messung im Szintillationszähler quantifiziert (Nr. 3, Figur 4). Es werden 2 µg des Proteins je ml Reaktionslösung erhalten. Die Homogenität des synthetisierten Proteins wurde gelelektrophoretisch analysiert (Figur 6).

Die zweite im Beispiel genannte mRNA wurde wie vorstehend angegeben translatiert und die Proteinausbeute quantifiziert (Nr. 4**,** Figur 4). Es werden 5,7 µg/ml Reaktionslösung des Proteins erhalten. Die Homogenität des synthetisierten Proteins wurde gelelektrophoretisch analysiert (Figur 6).

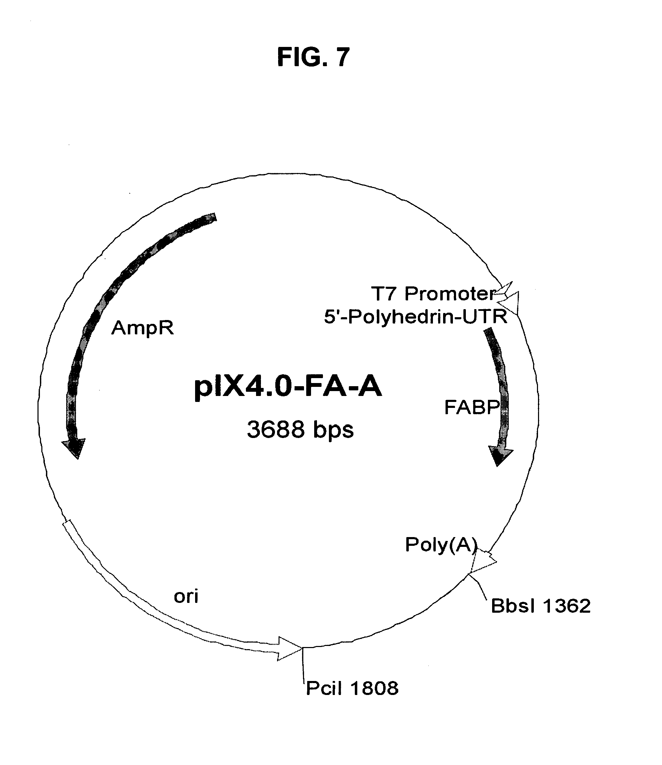

Für Nr. 1-2 wurde das eukaryontische Regulationselemente enthaltende Plasmid pIX4.0-FA-A (Figur 7, Seq.-ID 3) mit den Restriktionsenzymen BbsI und PciI verdaut, wie in Beispiel 2 angegeben aufgereinigt und die DNA-Konzentration bestimmt. Die DNA wurde wie in Beispiel 2 beschrieben für die Transkriptionsreaktion eingesetzt. Anstelle des NTP-Mix des Kits wurden folgende Komponenten mit den auf die Transkriptionsreaktion bezogenen Endkonzentrationen eingesetzt. Nr. 1: 3,75 mM jeweils ATP, CTP und UTP, 1,5 mM GTP, 0,5 mM Cap (m⁷G(5')ppp(5')G, Katalog-Nr. 8050, Ambion); Nr. 2: Komponenten wie bei Nr. 5. Die aus der Transkription des Plasmidfragments aus pIX4.0-FA-A resultierende RNA Nukleotidsequenz ist in Seq.-ID 4 angegeben. Alle mRNAs wurden gelelektrophoretisch analysiert (Figur 5).

Alle hier beschriebenen Transkriptionsansätze wurden wie in Beispiel 2, zweiter Teil, beschrieben für die Translationsreaktion eingesetzt und die jeweiligen Proteinausbeuten bestimmt. Für Nr. 6 wurde anstelle von RNA RNase-freies Wasser eingesetzt. Alle Proteinsynthesereaktionen wurden gelelektrophoretisch analysiert (Figur 6).

### Beispiel 3a : Expression der Proteine aus Beispiel 2 in einem eukaryontischen, auf Säugetierzellen basierenden System.

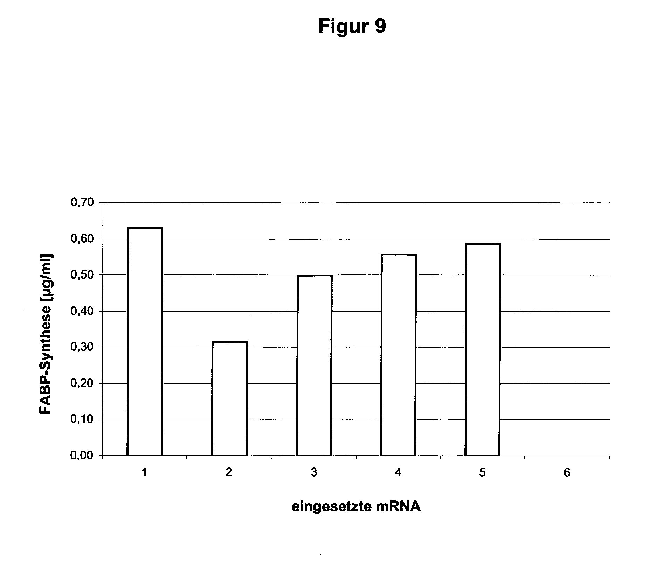

Die erste in Beispiel 2 beschriebene mRNA wurde bezogen auf die Translationsreaktion in einer Endkonzentration von 300 nM für die nach Herstellerangaben durchgeführte zellfreie Translation mit dem TNT Reticulocyte Lysate System (Katalog-Nr. L4610, Promega) eingesetzt. Zusätzlich wurde für die Translation ¹⁴C-markiertes Leucin eingesetzt, so dass sich eine molare Aktivität von 706 dpm/pmol eingestellt hat. Die Proteinausbeuten wurden anhand des Einbaus der radioaktiv markierten Aminosäure in heißer Trichloressigsäure unlöslichem Reaktionsprodukt und Messung im Szintillationszähler quantifiziert (Nr. 3, Fig. 9). Es werden 0,50 µg des Proteins je ml Reaktionslösung erhalten.

Die zweite im Beispiel genannte mRNA wurde wie vorstehend angegeben translatiert und die Proteinausbeute quantifiziert (Nr. 4**,** Fig. 9). Es werden 0,56 *µ*g des Proteins je ml Reaktionslösung erhalten.

### Beispiel 4: Expression des erstgenannten Proteins aus Beispiel 2 in einem prokaryontischen System.

Die RNA wurde bezogen auf die Translationsreaktion in einer Endkonzentration von 400 nM für die nach Herstellerangaben durchgeführte zellfreie Translation mit dem auf *Escherichia coli* Zellen basierendem *in-vitro*-PBS-Kit (Katalog-Nr. P-1102, RiNA GmbH, Berlin) eingesetzt. Zusätzlich wurde für die Translation ¹⁴C-markiertes Valin eingesetzt, so dass sich eine molare Aktivität von 3,35 dpm/pmol eingestellt hat.

Die Proteinausbeuten wurden anhand des Einbaus der radioaktiv markierten Aminosäure in heißer Trichloressigsäure unlöslichem Reaktionsprodukt und Messung im Szintillationszähler quantifiziert. Die Homogenität des synthetisierten Proteins wurde gelelektrophoretisch analysiert (Figur 6)

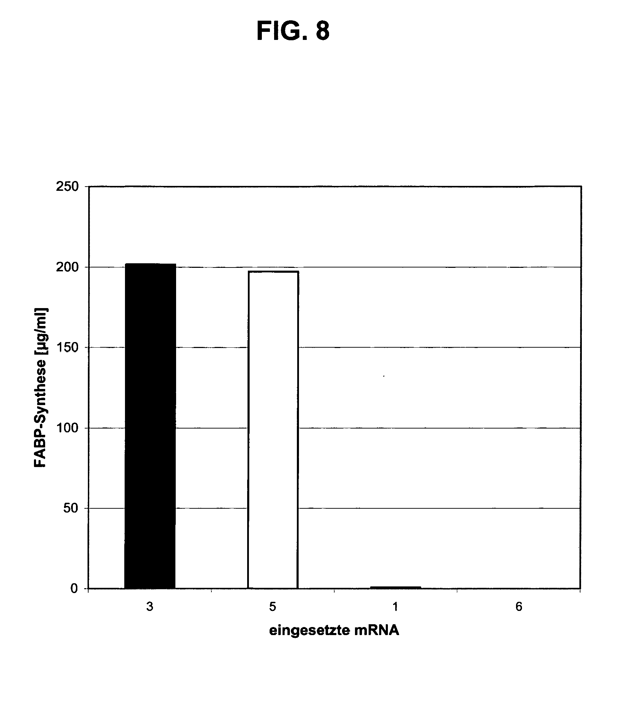

Es werden 200 µg/ml Reaktionslösung des Proteins erhalten (siehe Nr. 4, Figur 8).

### Beispiel 5: Ergebnisse

Figur 4 zeigt Proteinausbeuten in Abhängigkeit der verschiedenen für das Fettsäure Bindende Protein kodierenden mRNAs. Kontroll-mRNAs wurden wie in Beispiel 2 beschrieben hergestellt mit folgenden Abweichungen. Für Nr. 5 wurden anstelle des NTP-Mix des Kits folgende Komponenten mit den auf die Transkriptionsreaktion bezogenen Endkonzentrationen eingesetzt: 3,75 mM jeweils ATP, CTP und UTP, 1,5 mM GTP.

Die Figur 5 zeigt eine elektrophoretische Analyse der verschiedenen mRNAs. Von jeder mRNA wurden 200 ng auf das Agarosegel aufgetragen und das Gel nach der Auftrennung mit Ethidiumbromid gefärbt. Die Numerierung der Spuren entspricht der Numerierung der mRNAs in Abbildung 4. R: RNA Größenstandards; K: Kontroll-RNA. Eingesetzt wurden die folgenden mRNAs (Bahnnummer):
- Nr. 1:: eukaryontentypische mRNA mit Cap, effektiver insektenspezifischer 5'-UTR aus baculoviralem Polyhedringen und Poly(A)-Sequenz am 3'-Ende
- Nr. 2:: mRNA wie Nr. 1 ohne Cap
- Nr. 3:: erfindungsgemäße mRNA ohne Cap stattdessen mit Biotin am 5'-Ende, ohne insektenspezifische 5'-UTR und ohne Kozak-Sequenz stattdessen mit prokaryontentypischer 5' -UTR und starker Sekundärstruktur am 5'-Ende, ohne Poly(A)-Sequenz am 3'-Ende stattdessen mit prokaryontentypischer 3'-UTR mit T7 Pagen Transkriptionsterminator am 3'-Ende
- Nr. 4:: erfindungsgemäße mRNA wie Nr. 3 ohne Cap stattdessen mit P1,P3-Di(guanosine-5')triphosphate am 5'-Ende, ohne insektenspezifische 5'-UTR und ohne Kozak-Sequenz stattdessen mit prokaryontentypischer 5'-UTR und starker Sekundärstruktur am 5'-Ende, ohne 3'-Poly(A) stattdessen mit prokaryontentypischer 3'-UTR mit T7 Pagen Transkriptionsterminator am 3'-Ende
- Nr. 5:: mRNA wie Nr. 3 ohne Biotin
- Nr. 6:: ohne mRNA

Figur 6 zeigt die elektrophoretische Analyse der Homogenität des synthetisierten Proteins ausgehend von den verschiedenen mRNAs.

Die in den Beispielen 3 und 4 beschriebenen Proteinsyntheseansätze wurden im SDS-Polyacrylamidgel aufgetrennt und ein Autoradiogramm des Gels erstellt. e: Expression im eukaryontischen System, p: Expression im prokaryontischen System. Die Ziffern entsprechen der Numerierung in Abb. 4. M1: Markerprotein, M2: nichtradioaktive Markerproteine (unsichtbar im Autoradiogramm)

Wie aus Figur 4 hervorgeht, werden im verwendeten eukaryontischen zellfreien Translationssystem mit der erfindungsgemäßen mRNA mit Shine-Dalgarno Sequenz und starker Sekundärstruktur am 5'-Ende, ohne Cap am 5'-Ende, ohne Kozak-Sequenz, ohne die Translation effizient steigernder insektenspezifischer 5'-UTR und ohne Poly(A)-Sequenz am 3'-Ende (Nr. 3 und 4) mit 2 bzw. 5,7 µg/ml hohe Ausbeuten an Fettsäure Bindendem Protein im Vergleich zur eukaryontentypischen mRNA ohne Shine-Dalgarno Sequenz, mit Cap am 5'-Ende, mit insektenspezifischer 5'-UTR und Poly(A)-Sequenz am 3'-Ende (Nr. 1) erzielt.
Darüber hinaus wird die eukaryontentypische mRNA ohne Modifikation am 5'-Ende (Nr. 2) nur mit geringer Effizienz translatiert. Dies zeigt, dass die Expressionsstärke für diese mRNA stark von der Anwesenheit von Cap abhängt Im Gegensatz hierzu wird die prokaryontentypische mRNA ohne Modifikation am 5'-Ende (Nr. 5) etwa doppelt so effizient, die prokaryontentypische mRNA ohne Cap aber mit Biotin am 5'-Ende (Nr. 3) etwa dreimal so effizient und die prokaryontentypische mRNA ohne Cap aber mit P1,P3-Di(guanosine-5')triphosphate am 5'-Ende (Nr. 4) etwa zehnmal so effizient translatiert.

Figur 6 zeigt, dass das synthetisierte Fettsäurebindende Protein (14,8 kDa) mit dem erwarteten Molekulargewicht detektiert wird. Für die eukaryontentypische mRNA wird das synthetisierte Protein in Form von drei Banden detektiert (Spur e1). Dagegen erscheint das Protein im wesentlichen in Form von einer Bande, wenn die erfindungsgemäße mRNA als Matrize für die Proteinsynthese in eu- und prokaryontischem Proteinsynthesesystem eingesetzt wird (Spuren e3. e4, p3 und p4).

Figur 8 zeigt Proteinausbeuten im prokaryontischen System in Abhängigkeit der verschiedenen für das Fettsäure Bindende Protein kodierenden mRNAs. Zur Herstellung der mRNA (Nr. 3) und der Vergleichs-mRNAs (Nr. 1, 5 und 6) wird auf die Figur 4 und den begleitenden Text verwiesen. Wie aus Figur 8 hervorgeht, wird die erfindungsgemäße mRNA (Nr. 3) mit 200 *µ*g/ml Ausbeute an Fettsäure Bindendem Protein sehr effizient translatiert. Die Proteinausbeute ist praktisch genauso hoch wie bei Standard mRNA für dieses System, welche kein Biotin am 5'-Ende enthält (Nr. 4). Dagegen wird die eukaryontentypische mRNA mit Cap am 5'-Ende, mit Insektenspezifischer 5'-UTR und Poly(A)-Sequenz am 3'-Ende (Nr. 1) praktisch überhaupt nicht translatiert. Dies zeigt, dass das System spezifisch ist für mRNA mit Prokaryonten-typischen Elementen und dass die Modifikation der prokaryontischen mRNA am 5'-Ende keinen Einfluß auf die Translationseffizienz hat.

Wie aus der Figur 9, welche Ergebnisse zum Beispiel 3a darstellt, hervorgeht, werden im verwendeten eukaryontischen zellfreien Translationssystem basierend auf Säugerzellen mit der erfindungsgemäßen mRNA mit Shine-Dalgarno Sequenz und starker Sekundärstruktur am 5'-Ende, ohne Cap am 5'-Ende, ohne Kozak-Sequenz, ohne die Translation effizient steigernder Säugerzell-spezifischer 5'-UTR und ohne Poly(A)-Sequenz am 3'-Ende (Nr. 3, 4 und 5) mit 0,50, 0,56 bzw. 0,59 µg/ml hohe Ausbeuten an Fettsäure Bindendem Protein im Vergleich zur Eukaryonten-typischen mRNA (Nr. 1) erzielt.

Darüber hinaus wird die eukaryontentypische mRNA ohne Modifikation am 5'-Ende (Nr. 2) nur mit geringer Effizienz translatiert. Dies zeigt, dass die Expressionsstärke für diese mRNA stark von der Anwesenheit von Cap abhängt Im Gegensatz hierzu werden die erfindungsgemäßen prokaryontentypischen mRNAs
a) ohne Modifikation am 5'-Ende (Nr. 5),
b) ohne Cap aber mit Biotin am 5'-Ende (Nr. 3) und/oder
c) ohne Cap aber mit P1,P3-Di(guanosine-5')triphosphate am 5'-Ende (Nr. 4)
etwa doppelt so effizient translatiert wie die eukaryontentypische mRNA ohne Modifikation am 5'-Ende.

### Annex zu den Anmeldungsunterlagen - nachgereichtes Sequenzprotokoll

Seq.-ID 1:

1 gggagaccac aacggtttcc ctctagaaat aattttgttt aactttaaga aggagataaa
61 caatggtgga cgccttcgtg ggtacctgga agttagtgga cagcaagaat ttcgatgact
121 acatgaagtc actcggtgtc ggttttgcta ccaggcaggt gggcaatatg accaagccta
181 ccacaatcat cgaagtgaat ggggacacag tcatcataaa aacacaaagc accttcaaga
241 acacagagat cagcttcaag ctgggagtcg agttcgatga gaccacagca gatgacagga
301 aagtcaagtc catcgtgacg ctggatggcg gcaaacttgt ccacgtgcag aagtggaatg
361 gacaagagac atcacttgtg cgggagatgg ttgacggaaa actcattctg acactcaccc
421 acggcactgc agtttgcact cgtacttacg agaaacaggc ataataacta actaaccaag
481 atctgtaccc cttggggcct ctaaacgggt cttgaggggt tttttgcccg ggatccggct
541 gctaacaaag cccgaaagga agctgagttg gctgctgcca ccgctgagca ataactagca
601 taaccccttg gggcctctaa acgggtcttg aggggttttt tg

Seq.-ID 2:

1 tcgcgcgttt cggtgatgac ggtgaaaacc tctgacacat gcagctcccg gagacggtca
61 cagcttgtct gtaagcggat gccgggagca gacaagcccg tcagggcgcg tcagcgggtg
121 ttggcgggtg tcggggctgg cttaactatg cggcatcaga gcagattgta ctgagagtgc
181 accatatgcg gtgtgaaata ccgcacagat gcgtaaggag aaaataccgc atcaggcgcc
241 attcgccatt caggctgcgc aactgttggg aagggcgatc ggtgcgggcc tcttcgctat
301 tacgccagct ggcgaaaggg ggatgtgctg caaggcgatt aagttgggta acgccagggt
361 tttcccagtc acgacgttgt aaaacgacgg ccagtgccaa gcttgcatgc aaggagatgg
421 cgcccaacag tcccccggcc acggggcctg ccaccatacc cacgccgaaa caagcgctca
481 tgagcccgaa gtggcgagcc cgatcttccc catcggtgat gtcggcgata taggcgccag
541 caaccgcacc tgtggcgccg gtgatgccgg ccacgatgcg tccggcgtag aggatcgaga
601 tctatttagg tgacactata gaatacatcg attaatacga ctcactatag ggagaccaca
661 acggtttccc tctagaaata attttgttta actttaagaa ggagataaac aatggtggac
721 gccttcgtgg gtacctggaa gttagtggac agcaagaatt tcgatgacta catgaagtca
781 ctcggtgtcg gttttgctac caggcaggtg ggcaatatga ccaagcctac cacaatcatc
841 gaagtgaatg gggacacagt catcataaaa acacaaagca ccttcaagaa cacagagatc
901 agcttcaagc tgggagtcga gttcgatgag accacagcag atgacaggaa agtcaagtcc
961 atcgtgacgc tggatggcgg caaacttgtc cacgtgcaga agtggaatgg acaagagaca
1021 tcacttgtgc gggagatggt tgacgggaaa ctcattctga cactcaccca cggcactgca
1081 gtttgcactc gtacttacga gaaacaggca taataactaa ctaaccaaga tctgtacccc
1141 ttggggcctc taaacgggtc ttgaggggtt ttttgcccgg gatccggctg ctaacaaagc
1201 ccgaaaggaa gctgagttgg ctgctgccac cgctgagcaa taactagcat aaccccttgg
1261 ggcctctaaa cgggtcttga ggggtttttt gctgaaagga ggaactatat ccggatatcc
1321 acaggacggg tgtggtcgcc atgatcgcgt agtcgatagt ggctccaagt agcgaagcga
1381 gcaggactgg gcggcggcca aagcggtcgg acagtgctcc gagaacgggt gcgcatagaa
1441 attgcatcaa cgcatatagc gctagaattc gtaatcatgg tcatagctgt ttcctgtgtg
1501 aaattgttat ccgctcacaa ttccacacaa catacgagcc ggaagcataa agtgtaaagc
1561 ctggggtgcc taatgagtga gctaactcac attaattgcg ttgcgctcac tgcccgcttt
1621 ccagtcggga aacctgtcgt gccagctgca ttaatgaatc ggccaacgcg cggggagagg
1681 cggtttgcgt attgggcgct cttccgcttc ctcgctcact gactcgctgc gctcggtcgt
1741 tcggctgcgg cgagcggtat cagctcactc aaaggcggta atacggttat ccacagaatc
1801 aggggataac gcaggaaaga acatgtgagc aaaaggccag caaaaggcca ggaaccgtaa
1861 aaaggccgcg ttgctggcgt ttttccatag gctccgcccc cctgacgagc atcacaaaaa
1921 tcgacgctca agtcagaggt ggcgaaaccc gacaggacta taaagatacc aggcgtttcc
1981 ccctggaagc tccctcgtgc gctctcctgt tccgaccctg ccgcttaccg gatacctgtc
2041 cgcctttctc ccttcgggaa gcgtggcgct ttctcatagc tcacgctgta ggtatctcag
2101 ttcggtgtag gtcgttcgct ccaagctggg ctgtgtgcac gaaccccccg ttcagcccga
2161 ccgctgcgcc ttatccggta actatcgtct tgagtccaac ccggtaagac acgacttatc
2221 gccactggca gcagccactg gtaacaggat tagcagagcg aggtatgtag gcggtgctac
2281 agagttcttg aagtggtggc ctaactacgg ctacactaga aggacagtat ttggtatctg

2341 cgctctgctg aagccagtta ccttcggaaa aagagttggt agctcttgat ccggcaaaca
2401 aaccaccgct ggtagcggtg gtttttttgt ttgcaagcag cagattacgc gcagaaaaaa
2461 aggatctcaa gaagatcctt tgatcttttc tacggggtct gacgctcagt ggaacgaaaa
2521 ctcacgttaa gggattttgg tcatgagatt atcaaaaagg atcttcacct agatcctttt
2581 aaattaaaaa tgaagtttta aatcaatcta aagtatatat gagtaaactt ggtctgacag
2641 ttaccaatgc ttaatcagtg aggcacctat ctcagcgatc tgtctatttc gttcatccat
2701 agttgcctga ctccccgtcg tgtagataac tacgatacgg gagggcttac catctggccc
2761 cagtgctgca atgataccgc gagacccacg ctcaccggct ccagatttat cagcaataaa
2821 ccagccagcc ggaagggccg agcgcagaag tggtcctgca actttatccg cctccatcca
2881 gtctattaat tgttgccggg aagctagagt aagtagttcg ccagttaata gtttgcgcaa
2941 cgttgttgcc attgctacag gcatcgtggt gtcacgctcg tcgtttggta tggcttcatt
3001 cagctccggt tcccaacgat caaggcgagt tacatgatcc cccatgttgt gcaaaaaagc
3061 ggttagctcc ttcggtcctc cgatcgttgt cagaagtaag ttggccgcag tgttatcact
3121 catggttatg gcagcactgc ataattctct tactgtcatg ccatccgtaa gatgcttttc
3181 tgtgactggt gagtactcaa ccaagtcatt ctgagaatag tgtatgcggc gaccgagttg
3241 ctcttgcccg gcgtcaatac gggataatac cgcgccacat agcagaactt taaaagtgct
3301 catcattgga aaacgttctt cggggcgaaa actctcaagg atcttaccgc tgttgagatc
3361 cagttcgatg taacccactc gtgcacccaa ctgatcttca gcatctttta ctttcaccag
3421 cgtttctggg tgagcaaaaa caggaaggca aaatgccgca aaaaagggaa taagggcgac
3481 acggaaatgt tgaatactca tactcttcct ttttcaatat tattgaagca tttatcaggg
3541 ttattgtctc atgagcggat acatatttga atgtatttag aaaaataaac aaataggggt
3601 tccgcgcaca tttccccgaa aagtgccacc tgacgtctaa gaaaccatta ttatcatgac
3661 attaacctat aaaaataggc gtatcacgag gccctttcgt c

Seq.-ID 3:

1 tcgcgcgttt cggtgatgac ggtgaaaacc tctgacacat gcagctcccg gagacggtca
61 cagcttgtct gtaagcggat gccgggagca gacaagcccg tcagggcgcg tcagcgggtg
121 ttggcgggtg tcggggctgg cttaactatg cggcatcaga gcagattgta ctgagagtgc
181 accatatgcg gtgtgaaata ccgcacagat gcgtaaggag aaaataccgc atcaggcgcc
241 attcgccatt caggctgcgc aactgttggg aagggcgatc ggtgcgggcc tcttcgctat
301 tacgccagct ggcgaaaggg ggatgtgctg caaggcgatt aagttgggta acgccagggt
361 tttcccagtc acgacgttgt aaaacgacgg ccagtgccaa gcttgcatgc aaggagatgg
421 cgcccaacag tcccccggcc acggggcctg ccaccatacc cacgccgaaa caagcgctca
481 tgagcccgaa gtggcgagcc cgatcttccc catcggtgat gtcggcgata taggcgccag
541 caaccgcacc tgtggcgccg gtgatgccgg ccacgatgcg tccggcgtag aggatcgaga
601 tcctaatacg actcactata gggtctagat aagtatttta ctgttttcgt aacagttttg
661 taataaaaaa acctataaat ccatggtgga cgccttcgtg ggtacctgga agttagtgga
721 cagcaagaat ttcgatgact acatgaagtc actcggtgtc ggttttgcta ccaggcaggt
781 gggcaatatg accaagccta ccacaatcat cgaagtgaat ggggacacag tcatcataaa
841 aacacaaagc accttcaaga acacagagat cagcttcaag ctgggagtcg agttcgatga
901 gaccacagca gatgacagga aagtcaagtc catcgtgacg ctggatggcg gcaaacttgt
961 ccacgtgcag aagtggaatg gacaagagac atcacttgtg cgggagatgg ttgacggaaa
1021 actcattctg acactcaccc acggcactgc agtttgcact cgtacttacg agaaacaggc
1081 atgacctgcc ctctcctccc actgactgct cttctgtcag tgggctactc ctggactcgg
1141 caccagattg cctcattttt ctcctctggc attttgtata aatccacctt gactggggaa
1201 attctcctgg ggtcaggtgg caccagcctg gatctaaata ataagtaatt aactagttaa
1261 gtaaccaaga tccgtaagct agcacaaccg cgtctcaaaa aaaaaaaaaa aaaaaaaaaa
1321 aaaaaaaaaa aaaaaaaaaa aaaaaaaaaa aaaaaaaaac agtcttcgct gagcaataac
1381 tagcataacc ccttggggcc tctaaacggg tcttgagggg ttttttgata tcccggatt
1441 taaataggcc tgaattcgta atcatggtca tagctgtttc ctgtgtgaaa ttgttatccg
1501 ctcacaattc cacacaacat acgagccgga agcataaagt gtaaagcctg gggtgcctaa
1561 tgagtgagct aactcacatt aattgcgttg cgctcactgc ccgctttcca gtcgggaaac
1621 ctgtcgtgcc agctgcatta atgaatcggc caacgcgcgg ggagaggcgg tttgcgtatt
1681 gggcgctctt ccgcttcctc gctcactgac tcgctgcgct cggtcgttcg gctgcggcga
1741 gcggtatcag ctcactcaaa ggcggtaata cggttatcca cagaatcagg ggataacgca
1801 ggaaagaaca tgtgagcaaa aggccagcaa aaggccagga accgtaaaaa ggccgcgttg

1861 ctggcgtttt tccataggct ccgcccccct gacgagcatc acaaaaatcg acgctcaagt
1921 cagaggtggc gaaacccgac aggactataa agataccagg cgtttccccc tggaagctcc
1981 ctcgtgcgct ctcctgttcc gaccctgccg cttaccggat acctgtccgc ctttctccct
2041 tcgggaagcg tggcgctttc tcatagctca cgctgtaggt atctcagttc ggtgtaggtc
2101 gttcgctcca agctgggctg tgtgcacgaa ccccccgttc agcccgaccg ctgcgcctta
2161 tccggtaact atcgtcttga gtccaacccg gtaagacacg acttatcgcc actggcagca
2221 gccactggta acaggattag cagagcgagg tatgtaggcg gtgctacaga gttcttgaag
2281 tggtggccta actacggcta cactagaagg acagtatttg gtatctgcgc tctgctgaag
2341 ccagttacct tcggaaaaag agttggtagc tcttgatccg gcaaacaaac caccgctggt
2401 agcggtggtt tttttgtttg caagcagcag attacgcgca gaaaaaaagg atctcaagaa
2461 gatcctttga tcttttctac ggggtctgac gctcagtgga acgaaaactc acgttaaggg
2521 attttggtca tgagattatc aaaaaggatc ttcacctaga tccttttaaa ttaaaaatga
2581 agttttaaat caatctaaag tatatatgag taaacttggt ctgacagtta ccaatgctta
2641 atcagtgagg cacctatctc agcgatctgt ctatttcgtt catccatagt tgcctgactc
2701 cccgtcgtgt agataactac gatacgggag ggcttaccat ctggccccag tgctgcaatg
2761 ataccgcgag acccacgctc accggctcca gatttatcag caataaacca gccagccgga
2821 agggccgagc gcagaagtgg tcctgcaact ttatccgcct ccatccagtc tattaattgt
2881 tgccgggaag ctagagtaag tagttcgcca gttaatagtt tgcgcaacgt tgttgccatt
2941 gctacaggca tcgtggtgtc acgctcgtcg tttggtatgg cttcattcag ctccggttcc
3001 caacgatcaa ggcgagttac atgatccccc atgttgtgca aaaaagcggt tagctccttc
3061 ggtcctccga tcgttgtcag aagtaagttg gccgcagtgt tatcactcat ggttatggca
3121 gcactgcata attctcttac tgtcatgcca tccgtaagat gcttttctgt gactggtgag
3181 tactcaacca agtcattctg agaatagtgt atgcggcgac cgagttgctc ttgcccggcg
3241 tcaatacggg ataataccgc gccacatagc agaactttaa aagtgctcat cattggaaaa
3301 cgttcttcgg ggcgaaaact ctcaaggatc ttaccgctgt tgagatccag ttcgatgtaa
3361 cccactcgtg cacccaactg atcttcagca tcttttactt tcaccagcgt ttctgggtga
3421 gcaaaaacag gaaggcaaaa tgccgcaaaa aagggaataa gggcgacacg gaaatgttga
3481 atactcatac tcttcctttt tcaatattat tgaagcattt atcagggtta ttgtctcatg
3541 agcggataca tatttgaatg tatttagaaa aataaacaaa taggggttcc gcgcacattt
3601 ccccgaaaag tgccacctga cgtctaagaa accattatta tcatgacatt aacctataaa
3661 aataggcgta tcacgaggcc ctttcgtc

Seq.-ID 4:

1 gggtctagat aagtatttta ctgttttcgt aacagttttg taataaaaaa acctataaat
61 ccatggtgga cgccttcgtg ggtacctgga agttagtgga cagcaagaat ttcgatgact
121 acatgaagtc actcggtgtc ggttttgcta ccaggcaggt gggcaatatg accaagccta
181 ccacaatcat cgaagtgaat ggggacacag tcatcataaa aacacaaagc accttcaaga
241 acacagagat cagcttcaag ctgggagtcg agttcgatga gaccacagca gatgacagga
301 aagtcaagtc catcgtgacg ctggatggcg gcaaacttgt ccacgtgcag aagtggaatg
361 gacaagagac atcacttgtg cgggagatgg ttgacggaaa actcattctg acactcaccc
421 acggcactgc agtttgcact cgtacttacg agaaacaggc atgacctgcc ctctcctccc
481 actgactgct cttctgtcag tgggctactc ctggactcgg caccagattg cctcattttt
541 ctcctctggc attttgtata aatccacctt gactggggaa attctcctgg ggtcaggtgg
601 caccagcctg gatctaaata ataagtaatt aactagttaa gtaaccaaga tccgtaagct
661 agcacaaccg cgtctcaaaa aaaaaaaaaa aaaaaaaaaa aaaaaaaaaa aaaaaaaaaa
721 aaaaaaaaaa aaaaaaaaaa

## Patentansprüche

1. Verfahren zur Genexpression in einem zellfreien Translationssystem, wobei die Reaktionslösung eine RNA-Matrize mit einer Gensequenz, die für ein zu exprimierendes Expressionsprodukt kodiert, sowie ein Translationssystem aus eukaryontischen Zellen, enthält, wobei die Reaktionslösung inkubiert wird, und wobei das Expressionsprodukt optional von der Reaktionslösung abgetrennt wird, **dadurch gekennzeichnet, dass** die RNA-Matrize, in 5'- 3'- Richtung betrachtet, eine Shine Dalgarno Sequenz, hieran angeschlossen eine erste Spacer Sequenz und hieran angeschlossen die Gensequenz enthält.

2. Verfahren nach Anspruch 1, **dadurch gekennzeichnet,**
**dass** im 5'- Bereich der RNA-Matrize vor der Shine Dalgarno Sequenz kein ATG und/oder kein AUG mit open reading frame angeordnet sind, und/oder
**dass** das 5'-terminale Ende der Nukleotidsequenz der RNA-Matrize ein cap oder eine sterischen Schutzgruppe, insbesondere einem Biotinrest, aufweist, und/oder
**dass** zwischen dem 5'-terminalen Ende der RNA-Matrize und der Shine Dalgarno Sequenz eine Enhancersequenz, insbesondere eine von Bakteriophagen abstammende 5'-nicht-translatierte Sequenz (5'-UTR), angeordnet ist, und/oder
**dass** zwischen dem 5'-terminalen Ende der RNA-Matrize und der Enhancersequenz eine Hairpinstruktur angeordnet ist, und/oder
**dass** das 3'-terminale Ende der RNA-Matrize durch einen von Bakteriophagen abstammender Transkriptionsterminator, insbesondere durch eine Hairpinstruktur, gebildet wird und/oder
**dass** die erste Spacersequenz aus 3 bis 10, vorzugsweise 5 bis 8, Nukleotiden, vorzugsweise Pyrimidin-reichen Nukleotiden, gebildet ist, und/oder
**dass** zwischen der Gensequenz und dem 3'-terminalen Ende der RNA-Matrize eine zweite Spacersequenz angeordnet ist, welche vorzugsweise eine Länge von 10 bis 50, insbesondere 20 bis 40, Nukleotiden aufweist.

3. Verfahren nach einem der Ansprüche 1 oder 2, **dadurch gekennzeichnet, dass** die RNA-Matrize vom 5'-terminalen Ende beginnend die folgenden optional unmittelbar aneinander anschließenden Strukturelemente aufweist: Biotin, optional Hairpin, optional Enhancersequenz, Shine Dalgarno Sequenz, erste Spacersequenz, Gensequenz, optional zweite Spacersequenz, Transkriptionsterminator.

4. Verfahren nach einem der Ansprüche 1 bis 3, **dadurch gekennzeichnet, dass** das Translationssystem aus Insektenzellen gewonnen wurde.

5. Kit für die Genexpression in einem zellfreien Translationssystem, insbesondere für ein Verfahren nach einem der Ansprüche 1 bis 4, enthaltend die folgenden Komponenten:
a) Translationssystem aus eukaryontischen Zellen, insbesondere Insektenzellen,
b) RNA-Matrize, die, in 5'-3'-Richtung betrachtet, eine Shine Dalgarno Sequenz, hieran angeschlossen eine erste Spacer Sequenz und hieran angeschlossen eine Gensequenz enthält, oder DNA Vektor codierend für eine solche RNA.

6. Kit nach Anspruch 5, **dadurch gekennzeichnet,**
**dass** im 5'- Bereich der RNA-Matrize vor der Shine Dalgarno Sequenz kein ATG und/oder kein AUG mit open reading frame angeordnet sind, und/oder
**dass** das 5'-terminale Ende der Nukleotidsequenz der RNA-Matrize ein cap oder eine sterische Schutzgruppe, insbesondere einen Biotinrest, aufweist, und/oder
**dass** zwischen dem 5'-terminalen Ende der RNA-Matrize und der Shine Dalgarno Sequenz eine Enhancersequenz, insbesondere eine von Bakteriophagen abstammende 5'-nicht-translatierte Sequenz (5'-UTR) angeordnet ist, und/oder
**dass** zwischen dem 5'-terminalen Ende der RNA-Matrize und der Enhancersequenz eine Hairpinstruktur angeordnet ist, und/oder
**dass** das 3'-terminale Ende der RNA-Matrize durch einen von Bakteriophagen abstammenden Transkriptionsterminator, insbesondere durch eine Hairpinstruktur, gebildet wird, und/oder
**dass** die erste Spacersequenz aus 3 bis 10, vorzugsweise 5 bis 8, Nukleotiden, vorzugsweise Pyrimidin-reichen Nukleotiden, gebildet ist und/oder
**dass** zwischen der Gensequenz und dem 3'-terminalen Ende der RNA-Matrize eine zweite Spacersequenz angeordnet ist, welche vorzugsweise eine Länge von 10 bis 50, insbesondere 20 bis 40, Nukleotiden aufweist.

7. Kit nach einem der Ansprüche 5 oder 6, **dadurch gekennzeichnet, dass** die RNA-Matrize vom 5'-terminalen Ende beginnend die folgenden vorzugsweise unmittelbar aneinander anschließenden Strukturelemente aufweist: Biotin, optional Hairpin, Enhancersequenz, Shine Dalgarno Sequenz, erste Spacersequenz, Gensequenz, optional zweite Spacersequenz, Transkriptionsterminator.

8. Kit nach einem der Ansprüche 5 bis 7, **dadurch gekennzeichnet, dass** es als Komponente c) eine oder mehrere Stoffe aus der Gruppe bestehend aus "Aminosäuren, Energie liefernde und für die Synthese des Expressionsproduktes notwendige Stoffwechselbestandteile" enthält.

9. RNA enthaltend eine sterische Schutzgruppe, nicht jedoch ein cap, am 5'-terminalen Ende, eine Shine Dalgarno Sequenz und am 3'-Ende der Shine Dalgarno Sequenz eine Gensequenz, optional mit folgenden vorzugsweise unmittelbar aneinander anschließenden Strukturelementen, vom 5'-terminalen Ende beginnend: Biotin, optional Hairpin, optional Enhancersequenz, Shine Dalgarno Sequenz, erste Spacersequenz, Gensequenz, optional zweite Spacersequenz, Transkriptionsterminator.

10. DNA codierend für eine RNA nach Anspruch 9.
